# FASCICULE DE BREVET EUROPEEN

(11) **EP 1 592 394 B1**
(45) Date de publication et mention de la délivrance du brevet: **17.01.2007**
(21) Numéro de dépôt: 04707248.3
(22) Date de dépôt: 02.02.2004
(51) Int. Cl.: A61K 8/49, A61K 8/86, A61Q 5/06

(54) **COMPOSITION COSMETIQUE COMPRENANT UN AMIDE CATIONIQUE HYDROGELIFIANT ET UN TENSIOACTIF**
KOSMETISCHE ZUSAMMENSETZUNG ENTHALTEND EIN KATIONISCHES IN WASSER GEL-BILDENDES AMID UND EIN TENSID
COSMETIC COMPOSITION COMPRISING A HYDROGELLING CATIONIC AMIDE AND A DETERGENT

(30) Priorité: 03.02.2003 FR 0350011; 10.06.2003 US 477042 P
(43) Date de publication de la demande: 09.11.2005
(73) Titulaire: L'ORÉAL, 75008 Paris (FR)
(72) Inventeur: ROLLAT-CORVOL, Isabelle, F-75017 Paris (FR); BENABDILLAH, Katarina, F-92110 Clichy (FR)
(74) Mandataire: Bourdeau, Françoise
(86) Numéro de dépôt international: PCT/FR2004/000228
(87) Numéro de publication internationale: WO 2004/068998

(56) Documents cités:
- EP-A- 1 197 206
- WO-A-99/15610
- MASAHIRO SUZUKI ET AL.: "Low molecular-weight hydrogelators based on L-lysine with various pyridinium cations" CHEMISTRY LETTERS, 2002, pages 704-705, XP009018287 cité dans la demande
- MASAHIRO SUZUKI ET AL.: "New low-molecular-weight hydrogelators based on L-lysine with positively charged pendant chain" NEW JOURNAL OF CHEMISTRY, vol. 26, 2002, pages 817-818, XP009018282 cité dans la demande
- MASAHIRO SUZUKI ET AL.: "Novel family of low molecular weight hydrogelators based on L-lysine derivatives" CHEM.COMM., 2002, pages 884-885, XP009018628 cité dans la demande

## Description

L'invention a pour objet une composition cosmétique comprenant au moins un tensioactif et au moins un amide cationique hydrogélifiant. Elle vise également un procédé cosmétique comprenant l'application de cette composition, ainsi que son utilisation, en particulier dans le domaine capillaire.

On connaît des compositions épaissies, notamment des gels capillaires, qui sont généralement appliquées sur les cheveux avant de mettre en forme la coiffure. Pour mettre en forme et fixer la coiffure, on effectue ensuite un brushing ou une mise en plis. Ces compositions épaissies, notamment ces gels, peuvent contenir des résines polymères.

Toutefois, elles présentent souvent l'inconvénient d'altérer les propriétés cosmétiques des cheveux. Ainsi, les cheveux peuvent devenir rêches, difficiles à démêler, perdre leur toucher et leur aspect agréables ou encore manquer de corps. On recherche donc de telles compositions cosmétiques capillaires procurant de bonnes propriétés cosmétiques, notamment en terme de démêlage, de douceur et de toucher.

En outre, les compositions cosmétiques capillaires épaissies présentent également l'inconvénient majeur de donner lieu à un effet de poudrage. Au sens de la présente invention, on entend par « poudrage », l'aptitude du matériau obtenu par séchage de la composition capillaire à former une poudre après son application sur les cheveux.

Les milieux cosmétiques aqueux sont, en général, gélifiés par des polymères hydrophiles réticulés. Il est difficile de réaliser des gels cosmétiques aqueux qui auraient, après le séchage sur les cheveux, une bonne résistance à un climat humide.

Il existe donc un besoin de trouver des compositions cosmétiques, notamment capillaires épaissies, notamment des gels capillaires, qui ne présentent pas l'ensemble des inconvénients indiqués ci-dessus, et qui, en particulier résistent à un climat humide.

Pour résoudre ce problème, l'invention propose l'utilisation en cosmétique de petites molécules chirales à fonctions amides et à chaînes grasses, contenant des fonctions cationiques. Ces molécules sont décrites dans la littérature, et notamment dans les publications suivantes: Chemistry Letters 2002, (7), 704-705; New Journal of Chemistry 2002, 26 (7), 817-818; Chemical Communications (Cambridge, United Kingdom) 2002, (8), 884-885, en association avec au moins un tensioactif.

La Demanderesse a maintenant découvert qu'après le séchage sur les cheveux, ces gels forment des dépôts ayant une excellente tenue à l'humidité.

L'invention a pour objet une composition cosmétique, comprenant, en milieu aqueux ou eau+solvant, au moins un tensioactif et au moins un composé moléculaire épaississant cationique non phosphoré, de poids moléculaire inférieur à 2 000 g/mol et comprenant au moins:
- deux groupements amide,
- un carbone asymétrique,
- une chaîne grasse comportant au moins huit carbones.

Par *"composé moléculaire",* on entend, au sens de la présente invention, tout composé dont le squelette ne résulte pas d'une polymérisation. Ceci n'exclut pas que le composé puisse contenir un ou plusieurs groupement polyoxyalkylénés ou polyoxyglycérilés.

Par *"molécule cationique",* on entend, au sens de la présente invention, une molécule comprenant au moins un atome d'azote quaternisé.

### COMPOSE MOLECULAIRE EPAISSISSANT

Conformément à l'invention, le composé moléculaire épaississant cationique, non phosphoré, est de poids moléculaire inférieur à 2 000 g/mol et il comprend au moins:
- deux groupements amide,
- un carbone asymétrique,
- une chaîne grasse comportant au moins huit carbones.

Plus avantageusement encore, la chaîne grasse comporte entre 8 et 30 atomes de carbone, et plus préférentiellement entre 8 et 22 atomes de carbone.

De préférence, le composé moléculaire épaississant est de poids moléculaire inférieur à 1000g/mol, et de préférence, inférieur à 800 g/mol.

Avantageusement, au moins une chaîne grasse est liée directement au groupement amide, et en particulier, au moins une chaîne grasse est liée au groupement amide par une fonction carbonyle. Plus préférentiellement, le composé moléculaire épaississant contient au moins deux chaînes grasses et, encore plus préférentiellement, au moins une chaîne grasse fait partie d'un groupement ester.

Selon on mode préféré de l'invention, le composé moléculaire épaississant contient au moins deux enchaînements -CONH-.

En particulier, le composé épaississant est présent dans le milieu aqueux à une concentration en poids comprise entre 0,1 et 60 %, et de préférence entre 0,25 et 25 % en poids, par rapport au poids total de la composition cosmétique.

Préférentiellement, la composition forme un film après séchage. Dans un mode préféré, le film est insoluble dans l'eau pure à température ambiante. Il peut, par contre, être soluble, à pH acide ou alcalin.

En tant que composé moléculaire épaississant, on peut citer le bromure de 1-(11-(((1S)-1-éthoxycarbonyl-5-((1-oxododécyl)amino)pentyl)amino)-11-oxoundécyl) pyridinium.

### TENSIOACTIF

Les tensioactifs peuvent être des tensioactifs anioniques, non-ioniques, amphotères ou un mélange de ces derniers.

A titre de tensioactifs anioniques utilisables, seuls ou en mélanges, dans le cadre de la présente invention, on peut citer notamment les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les sulfates d'alkyle, les alkyléther-sulfates, les alkylamidoéthersulfates, les alkylarylpolyéthersulfates, les monoglycéride-sulfates ; les alkylsulfonates, les phosphates d'alkyle, les alkylamidesulfonates, les alkylarylsulfonates, les α-oléfine-sulfonates, les paraffine-sulfonates ; les sulfosuccinates d'alkyle, les alkyléthersulfosuccinates, les alkylamidesulfosuccinates ; les sulfoacétates d'alkyle ; les acylsarcosinates ; et les acylglutamates, les groupes alkyle ou acyle de tous ces composés comportant de 6 à 24 atomes de carbone et le groupe aryle désignant de préférence un groupe phényle ou benzyle. On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les sulfosuccinamates d'alkyle, les iséthionates d'acyle et les N-acyltaurates, le groupe alkyle ou acyle de tous ces composés comportant de 12 à 20 atomes de carbone. Parmi les tensioactifs anioniques encore utilisables, on peut également citer les lactylates d'acyle dont le groupe acyle comporte de 8 à 20 atomes de carbone.

En outre, on peut encore citer les acides d'alkyl-D-galactoside uroniques et leurs sels ainsi que les acides alkyl(C₆-C₂₄)éther-carboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Parmi les tensioactifs anioniques cités ci-dessus, on préfère utiliser selon l'invention les sels de sulfates d'alkyle, d'alkyléther-sulfates et d'alkyléthercarboxylates, et leurs mélanges.

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)aminopropylmorpholine ; et leurs mélanges.

Parmi les tensioactifs non-ioniques cités ci-dessus, on utilise de préférence les alkyl(C₆-C₂₄)polyglycosides.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant, au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes ; et leurs mélanges.

Parmi les dérivés d'amines, on peut citer les produits commercialisés sous la dénomination MIRANOL^{®}, tels que décrits dans les brevets US-2 528 378 et US-2 781 354 et classés dans le dictionnaire CTFA, 3^{ème} édition, 1982, sous les dénominations Amphocarboxy-glycinate et Amphocarboxypropionate de structures respectives (2) et (3) :

R₂-CONHCH₂CH₂-N⁺(R₃)(R₄)(CH₂COO⁻) (2)

dans laquelle :
R₂ représente un groupe alkyle dérivé d'un acide R₂-COOH présent dans l'huile de coprah hydrolysée, un groupe heptyle, nonyle ou undécyle,
R₃ représente un groupe bêta-hydroxyéthyle, et
R₄ représente un groupe carboxyméthyle ;
et

R_{2'}-CONHCH₂CH₂-N(B)(C) (3)

dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
R₂, représente un groupe alkyle d'un acide Rg -COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé sous la dénomination commerciale MIRANOL^{®} C2M concentré par la société RHODIA.

Parmi les tensioactifs amphotères, on utilise de préférence les alkyl(C₈-C₂₀)bétaïnes, les alkyl(C₈-C₂₀)amidoalkyl(C₆-C₈)bétaïnes, les alkylamphodiacétates et leurs mélanges.

Les tensioactifs décrits ci-dessus peuvent être utilisés seuls ou en mélanges et leur quantité totale est, de préférence comprise entre 0,1 % et 30 % en poids, mieux encore entre 1 % à 25 % en poids et au mieux entre 4 % et 20 % en poids par rapport au poids total de la composition.

Le milieu aqueux cosmétiquement acceptable peut être constitué uniquement par de l'eau ou par un mélange d'eau et d'un solvant cosmétiquement acceptable tel qu'un alcool inférieur en C₁-C₄, tel que l'éthanol, l'isopropanol, le tertio-butanol, le n-butanol ; les alkylèneglycols comme le propylèneglycol, les éthers de glycol.

L'homme de métier veillera à choisir les éventuels additifs et leur quantité de manière à ce qu'ils ne nuisent pas aux propriétés intéressantes des compositions de la présente invention.

Le pH des compositions de l'invention est compris entre 4 et 8, de préférence entre 5 et 7.

Les compositions selon l'invention peuvent également contenir des additifs tels que des épaississants polymériques naturels ou synthétiques, anioniques, amphotères, zwitteroniques, non ioniques ou cationiques, associatifs ou non, des épaississants non polymériques comme des acides ou des électrolytes, des nacrants, des opacifiants, des solvants organiques, des parfums, des huiles minérales, végétales et/ou synthétiques, des esters d'acides gras, des colorants, des silicones volatiles ou non, organomodifiées ou non, cycliques ou acycliques, ramifiées ou non, des particules minérales ou organiques, des conservateurs, des agents de stabilisation du pH.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions peuvent se présenter sous forme de liquides fluides ou épaissis, de gels, de crèmes, de mousses, d'émulsions E/H, H/E ou d'émulsions multiples.

Les compositions de l'invention peuvent être appliquées sur les cheveux, la peau, les cils, les sourcils et les ongles.

Elles peuvent être utilisées, par exemple, comme shampooings, soins rincés ou non rincés, masques de soin profond, lotions ou crèmes de traitement du cuir chevelu et de la peau, démaquillants.

On les emploie tout particulièrement pour les cheveux.

Il existe différents modes d'application des compositions sur les cheveux. On peut citer ceux-ci:
Application 1 : la composition est appliquée sur les cheveux sous forme de gel ou de solution dans une composition capillaire contenant de l'eau, de l'alcool ou un autre solvant cosmétiquement acceptable ou leur mélange.
Application 2 : la composition est appliquée sur les cheveux sous forme de solution dans une composition capillaire contenant un ou plusieurs solvants cosmétiquement acceptables. L'eau est appliquée en deuxième temps, pour former un gel.
Application 3 : la composition est appliquée sur les cheveux sous forme de gel ou de solution dans une composition capillaire contenant de l'eau, de l'alcool ou un autre solvant cosmétiquement acceptable ou leur mélange. Après séchage, le dépôt formé sur les cheveux est éliminé par application:
   - d'eau chaude
   - d'une solution aqueuse à pH acide ou basique
   - d'un solvant ou d'un mélange de plusieurs solvants.

La présente invention concerne également un procédé de traitement cosmétique des matières kératiniques qui consiste à appliquer sur les matières kératiniques une quantité efficace d'une composition telle que décrite ci-dessus, et à effectuer éventuellement un rinçage après un éventuel temps de pause.

De préférence, les matières kératiniques sont des cheveux.

Les compositions peuvent être conditionnées sous forme d'aérosol.

L'invention concerne également l'utilisation sur cheveux pour obtenir un effet coiffant résistant à l'humidité d'une composition cosmétique selon l'invention.

### EXEMPLE:

On réalise la mousse de coiffage suivante :

| | |
|---|---|
| 1-(11-(((1S)-1-éthoxycarbonyl-5-((1-oxododécyl)amino)pentyl)amino)-11-oxoundécyl) pyridinium | 0.4% m.a. |
| monolaurate de sorbitane oxyéthyléné (20 OE) | 0.2 % m.a. |
| eau | 94.5% m.a. |
| isobutane/propane (85/15) | 5% m.a. |

Après l'application sur cheveux, on a remarqué que cette mousse donne une tenue à l'humidité supérieure à celle de la mousse suivante :

| | |
|---|---|
| Carbopol Ultrez 10 ® (Noveon) | 0.4% m.a. |
| monolaurate de sorbitane oxyéthyléné (20 OE) | 0.1% m.a. |
| eau | 94.5% m.a. |
| isobutane/propane (85/15) | 5% m.a. |

## Revendications

1. Composition cosmétique comprenant, en milieu aqueux ou eau+solvant, au moins un tensioactif et au moins un composé moléculaire épaississant cationique non phosphoré, de poids moléculaire inférieur à 2 000 g/mol, ce composé moléculaire comprenant au moins:
- deux groupements amide,
- un carbone asymétrique,
- une chaîne grasse comportant au moins huit carbones,
par molécule cationique, on entend une molécule comprenant au moins un atome d'azote quaternisé.

2. Composition selon la revendication 1, **caractérisée par le fait que** le composé moléculaire épaississant contient au moins deux enchaînements -CONH-.

3. Composition selon l'une quelconque des revendications précédentes, **caractérisée par le fait que** la chaîne grasse comporte entre 8 et 30 atomes de carbone, et plus préférentiellement entre 8 et 22 atomes de carbone.

4. Composition selon l'une quelconque des revendications précédentes, **caractérisée par le fait que** le composé moléculaire épaississant est de poids moléculaire inférieur à 1000g/mol, et de préférence, inférieur à 800 g/mol.

5. Composition selon l'une quelconque des revendications précédentes, **caractérisée par le fait qu'**au moins une chaîne grasse est liée directement au groupement amide.

6. Composition selon l'une quelconque des revendications 1 à 4, **caractérisée par le fait qu'**au moins une chaîne grasse est liée au groupement amide par une fonction carbonyle.

7. Composition selon l'une quelconque des revendications précédentes, **caractérisée par le fait que** le composé moléculaire épaississant contient au moins deux chaînes grasses.

8. Composition selon l'une quelconque des revendications précédentes, **caractérisée par le fait qu'**au moins une chaîne grasse fait partie d'un groupement ester.

9. Composition selon l'une quelconque des revendications précédentes, **caractérisée par le fait que** le composé épaississant est présent dans le milieu aqueux à une concentration en poids comprise entre 0,1 et 60 %, et de préférence entre 0,25 et 25 % en poids, par rapport au poids total de la composition cosmétique.

10. Composition selon l'une quelconque des revendications prêcédentes, **caractérisée par le fait que** la composition forme un film après séchage.

11. Composition selon la revendication 10, **caractérisée par le fait que** le film est insoluble dans l'eau pure à la température ambiante.

12. Composition selon la revendication 10, **caractérisée par le fait que** le film est soluble à pH acide ou alcalin.

13. Composition selon l'une quelconque des revendications précédentes, **caractérisée en ce que** les tensioactifs sont choisis parmi les tensioactifs anioniques, non-ioniques, amphotères et leurs mélanges.

14. Composition selon la revendication 13, **caractérisée en ce que** les tensioactifs anioniques sont choisis parmi les sels des composés suivants : les sulfates d'alkyle, les alkyléthersulfates, les alkylamidoéthersulfates, les alkylarylpolyéthersulfates, les monoglycéride-sulfates, les alkylsulfonates, les phosphates d'alkyle, les alkylamidesulfonates, les alkylarylsulfonates, les α-oléflne-sulfonates, les paraffine-sulfonates ; les sulfosuccinates d'alkyle, les alkyléthersulfosuccinates, les alkylamidesulfosuccinates ; les sulfo-acétates d'alkyle ; les acylsarcosinates ; et les acylglutamates ; les groupes alkyle ou acyle de ces composés comportant de 6 à 24 atomes de carbone et le groupe aryle désignant de préférence un groupe phényle ou benzyle ; les esters d'alkyle en C₆-C₂₄ et d'acides polyglycoside-carboxyliques ; les sulfosuccinamates d'alkyle, les iséthionates d'acyle et les N-acyltaurates, le groupe alkyle ou acyle comportant de 12 à 20 atomes de carbone ; les lactylates d'acyle dont le groupe acyle comporte de 8 à 20 atomes de carbone ; les acides d'alkyl-D-galactoside uroniques et leurs sels, les acides alkyl(C₆-C₂₄)éther-carboxyliques polyoxyalkylénés, les acides alkyl(C₆-C₂₄)-aryl(C₆-C₂₄)éther-carboxyliques polyoxyalkylénés, les acides alkyl(C₆-C₂₄)amidoéther carboxyliques polyoxyalkylénés et leurs sels ; et leurs mélanges.

15. Composition selon la revendication 14, **caractérisée en ce que** les tensioactifs anioniques sont choisis parmi les sels de sulfates d'alkyle, d'alkyléthersulfates et d'alkyléthercarboxylates, et leurs mélanges.

16. Composition selon la revendication 13, **caractérisée en ce que** les tensioactifs non-ioniques sont choisis parmi les alcools, les alpha-diols, les allyl(C₁-C₂₀)phénols ou les acides gras en C₈-C₁₈ polyéthoxylés, polypropoxylés ou polyglycérolés, le nombre de groupements oxyde d'éthylène ou oxyde de propylène allant de 2 à 50 et le nombre de groupements glycérol allant de 2 à 30, les copolymères d'oxyde d'éthylène et de propylène, les condensats d'oxyde d'éthylène et de propylène sur des alcools gras ; les amides gras polyéthoxylés ayant de 2 à 30 moles d'oxyde d'éthylène, les amides gras polyglycérolés comportant en moyenne 1 à 5 groupements glycérol ; les amines grasses polyéthoxylées ayant de 2 à 30 moles d'oxyde d'éthylène ; les esters d'acides gras du sorbitane éthoxylés ayant de 2 à 30 moles d'oxyde d'éthytène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines ; et leurs mélanges.

17. Composition selon la revendication 13, **caractérisée en ce que** les tensioactifs amphotères sont choisis parmi des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique hydrosolubilisant tel qu'un groupe carboxylate, sulfonate, sulfate, phosphate ou phosphonate ; les alkyl(C₈-C₂₀)bétaïnes, les sulfobétaines, les alkyl(C₈-C₂₀)amidoalkyl(C₆-C₈)bétaïnes ou les alkyl(C₈-C₂₀)amidoalkyl (C₆-C₉)sulfobétaïnes ; et leurs mélanges.

18. Composition selon l'une quelconque des revendications précédentes, **caractérisée en ce que** la quantité totale de tensioactif(s) est comprise entre 0,1 % et 30 % en poids, mieux encore entre 1 % à 25 % en poids et au mieux entre 4 % et 20 % en poids par rapport au poids total de la composition.

19. Composition selon l'une quelconque des revendications précédentes, **caractérisée en ce qu'**elle comprend en outre des additifs tels que des épaississants polymériques naturels ou synthétiques, anioniques, amphotères, zwitteroniques, non ioniques ou cationiques, associatifs ou non, des épaississants non polymériques comme des acides ou des électrolytes, des nacrants, des opacifiants, des solvants organiques, des parfums, des huiles minérales, végétales et/ou synthétiques, des esters d'acides gras, des colorants, des silicones volatiles ou non, organomodifiées ou non, cycliques ou acycliques, ramifiées ou non, des particules minérales ou organiques, des conservateurs, des agents de stabilisation du pH.

20. Procédé de traitement cosmétique des matières kératiniques, **caractérisé en ce que** l'on applique sur les matières kératiniques une composition cosmétique comprenant, en milieu aqueux ou eau+solvant, au moins un tensioactif pour la chevelure et au moins un composé moléculaire épaississant cationique non phosphoré, de poids moléculaire inférieur à 2 000 g/mol, ce composé moléculaire comprenant au moins:
- deux groupements amide,
- un carbone asymétrique,
une chaîne grasse comportant au moins huit carbones, et que l'on effectue éventuellement un rinçage après un éventuel temps de pause;
par molécule cationique, on entend une molécule comprenant au moins un atome d'azote quaternisé.

21. Procédé selon la revendication 20, **caractérisé par le fait que** les matières kératiniques sont des cheveux.

22. Utilisation sur cheveux pour obtenir un effet coiffant résistant à l'humidité d'une composition cosmétique comprenant, en milieu aqueux ou eau+solvant, au moins un tensioactif et au moins un composé moléculaire épaississant cationique non phosphoré, de poids moléculaire inférieur à 2 000 g/mol, ce composé moléculaire comprenant au moins:
- deux groupements amide,
- un carbone asymétrique,
- une chaîne grasse comportant au moins huit carbones;
par molécule cationique, on entend une molécule comprenant au moins un atome d'azote quaternisé.

## Claims

1. Cosmetic composition comprising, in an aqueous or water + solvent medium, at least one surfactant and at least one non-phosphorus-containing cationic molecular thickening compound with a molecular weight below 2000 g/mol, this molecular compound comprising at least:
- two amide groups,
- one asymmetric carbon, and
- one fatty chain containing at least eight carbons,
"cationic molecule" being understood as meaning a molecule comprising at least one quaternized nitrogen atom.

2. Composition according to Claim 1, **characterized in that** the molecular thickening compound contains at least two -CONH- linkages.

3. Composition according to either one of the preceding claims, **characterized in that** the fatty chain contains between 8 and 30 carbon atoms and particularly preferably between 8 and 22 carbon atoms.

4. Composition according to any one of the preceding claims, **characterized in that** the molecular thickening compound has a molecular weight below 1000 g/mol and preferably below 800 g/mol.

5. Composition according to any one of the preceding claims, **characterized in that** at least one fatty chain is bonded directly to the amide group.

6. Composition according to any one of Claims 1 to 4, **characterized in that** at least one fatty chain is bonded to the amide group via a carbonyl group.

7. Composition according to any one of the preceding claims, **characterized in that** the molecular thickening compound contains at least two fatty chains.

8. Composition according to any one of the preceding claims, **characterized in that** at least one fatty chain forms part of an ester group.

9. Composition according to any one of the preceding claims, **characterized in that** the thickening compound is present in the aqueous medium at a concentration of between 0.1 and 60% by weight and preferably of between 0.25 and 25% by weight, based on the total weight of the cosmetic composition.

10. Composition according to any one of the preceding claims, **characterized in that** it forms a film after drying.

11. Composition according to Claim 10, **characterized in that** the film is insoluble in pure water at room temperature.

12. Composition according to Claim 10, **characterized in that** the film is soluble at acidic or alkaline pH.

13. Composition according to any one of the preceding claims, **characterized in that** the surfactants are selected from anionic, non-ionic and amphoteric surfactants and mixtures thereof.

14. Composition according to Claim 13, **characterized in that** the anionic surfactants are selected from the salts of the following compounds: alkylsulphates, alkylethersulphates, alkylamidoethersulphates, alkylarylpolyethersulphates, monoglyceridesulphates, alkylsulphonates, alkylphosphates, alkylamidesulphonates, alkylarylsulphonates, α-olefinsulphonates, paraffinsulphonates; alkylsulphosuccinates, alkylethersulphosuccinates, alkylamidesulphosuccinates; alkylsulphoacetates; acylsarcosinates; and acylglutamates, the alkyl or acyl groups of these compounds containing from 6 to 24 carbon atoms and the aryl group preferably being a phenyl or benzyl group; C₆-C₂₄-alkyl esters of polyglycosidecarboxylic acids; alkylsulphosuccinamates, acylisethionates and N-acyltaurates, the alkyl or acyl group containing from 12 to 20 carbon atoms; acyllactylates in which the acyl group contains from 8 to 20 carbon atoms; alkyl-D-galactosideuronic acids and their salts, polyalkoxylated alkyl(C₆-C₂₄)ethercarboxylic acids, polyalkoxylated alkyl (C₆-C₂₄) aryl-(C₆-C₂₄)ethercarboxylic acids, polyalkoxylated alkyl(C₆-C₂₄)amidoethercarboxylic acids and their salts; and mixtures thereof.

15. Composition according to Claim 14, **characterized in that** the anionic surfactants are selected from the salts of alkylsulphates, alkylethersulphates and alkylethercarboxylates, and mixtures thereof.

16. Composition according to Claim 13, **characterized in that** the non-ionic surfactants are selected from polyethoxylated, polypropoxylated or polyglycerolated alcohols, alpha-diols, alkyl(C₁-C₂₀)phenols and C₈-C₁₈ fatty acids, the number of ethylene oxide or propylene oxide groups ranging from 2 to 50 and the number of glycerol groups ranging from 2 to 30; ethylene oxide/ propylene oxide copolymers, condensation products of ethylene oxide and propylene oxide with fatty alcohols; polyethoxylated fatty amides having from 2 to 30 mol of ethylene oxide, polyglycerolated fatty amides containing an average of 1 to 5 glycerol groups; polyethoxylated fatty amines having from 2 to 30 mol of ethylene oxide; ethoxylated fatty acid esters of sorbitan having from 2 to 30 mol of ethylene oxide; fatty acid esters of sucrose, fatty acid esters of polyethylene glycol, alkyl(C₆-C₂₄)polyglycosides, N-alkyl(C₆-C₂₄)glucamine derivatives, amine oxides; and mixtures thereof.

17. Composition according to Claim 13, **characterized in that** the amphoteric surfactants are selected from secondary or tertiary aliphatic amine derivatives in which the aliphatic group is a linear or branched chain containing from 8 to 22 carbon atoms and at least one hydrosolubilizing anionic group such as a carboxylate, sulphonate, sulphate, phosphate or phosphonate group; alkyl(C₈-C₂₀) betaines, sulphobetaines, alkyl (C₈-C₂₀) - amidoalkyl (C₆-C₈) betaines or alkyl (C₈-C₂₀)amidoalkyl (C₆-C₈) sulphobetaines; and mixtures thereof.

18. Composition according to any one of the preceding claims, **characterized in that** the total amount of surfactant(s) is between 0.1% and 30% by weight, preferably between 1% and 25% by weight and particularly preferably between 4% and 20% by weight, based on the total weight of the composition.

19. Composition according to any one of the preceding claims, **characterized in that** it also comprises additives such as associative or non-associative, natural or synthetic, anionic, amphoteric, zwitterionic, non-ionic or cationic polymeric thickeners, non-polymeric thickeners such as acids or electrolytes, pearlescent agents, opacifiers, organic solvents, perfumes, mineral, vegetable and/or synthetic oils, fatty acid esters, colourants, organomodified or non-organomodified, volatile or non-volatile, branched or unbranched cyclic or acyclic silicones, mineral or organic particles, preservatives and pH stabilizers.

20. Method for the cosmetic treatment of a keratin material, **characterized in that** a cosmetic composition comprising, in an aqueous or water + solvent medium, at least one surfactant for the hair and at least one non-phosphorus-containing cationic molecular thickening compound with a molecular weight below 2000 g/mol, this molecular compound comprising at least:
- two amide groups,
- one asymmetric carbon, and
- one fatty chain containing at least eight carbons,
"cationic molecule" being understood as meaning a molecule comprising at least one quaternized nitrogen atom, is applied to the keratin material and the latter is rinsed, if necessary, optionally after a waiting period.

21. Method according to Claim 20, **characterized in that** the keratin material is hair.

22. Use on hair, to obtain a moisture-resistant styling effect, of a composition comprising, in an aqueous or water + solvent medium, at least one surfactant and at least one non-phosphorus-containing cationic molecular thickening compound with a molecular weight below 2000 g/mol, this molecular compound comprising at least:
- two amide groups,
- one asymmetric carbon, and
- one fatty chain containing at least eight carbons,
"cationic molecule" being understood as meaning a molecule comprising at least one quaternized nitrogen atom.

## Patentansprüche

1. Kosmetische Zusammensetzung, die in einem wässrigen Medium oder Wasser + Lösungsmittel mindestens einen grenzflächenaktiven Stoff und mindestens eine nicht phosphorhaltige, kationische, verdickende molekulare Verbindung mit einer Molmasse unter 2 000 g/mol enthält, wobei die molekulare Verbindung zumindest aufweist:
- zwei Amidgruppen,
- ein asymmetrisches Kohlenstoffatom,
- eine Fettkette mit mindestens acht Kohlenstoffatomen;
wobei unter einem kationischen Molekül ein Molekül verstanden wird, das mindestens ein quaternisiertes Stickstoffatom aufweist.

2. Zusammensetzung nach Anspruch 1, **dadurch gekennzeichnet, dass** die verdickende molekulare Verbindung mindestens zwei Verknüpfungen -CONH- aufweist.

3. Zusammensetzung nach einem der vorhergehenden Ansprüche, **dadurch gekennzeichnet, dass** die Fettkette 8 bis 30 Kohlenstoffatome und bevorzugt 8 bis 22 Kohlenstoffatome enthält.

4. Zusammensetzung nach einem der vorhergehenden Ansprüche, **dadurch gekennzeichnet, dass** die verdickende molekulare Verbindung eine Molmasse unter 1 000 g/mol und vorzugsweise unter 800 g/mol besitzt.

5. Zusammensetzung nach einem der vorhergehenden Ansprüche, **dadurch gekennzeichnet, dass** mindestens eine Fettkette direkt an eine Amidgruppe gebunden ist.

6. Zusammensetzung nach einem der Ansprüche 1 bis 4, **dadurch gekennzeichnet, dass** mindestens eine Fettkette über eine Carbonylfunktion an eine Amidgruppe gebunden ist.

7. Zusammensetzung nach einem der vorhergehenden Ansprüche, **dadurch gekennzeichnet, dass** die verdickende molekulare Verbindung mindestens zwei Fettketten enthält.

8. Zusammensetzung nach einem der vorhergehenden Ansprüche, **dadurch gekennzeichnet, dass** mindestens eine Fettkette Teil einer Estergruppe ist.

9. Zusammensetzung nach einem der vorhergehenden Ansprüche, **dadurch gekennzeichnet, dass** die verdickende Verbindung in dem wässrigen Medium in einer Konzentration von 0,1 bis 60 Gew.-% und vorzugsweise 0,25 bis 25 Gew.-%, bezogen auf das Gesamtgewicht der kosmetischen Zusammensetzung, enthalten ist.

10. Zusammensetzung nach einem der vorhergehenden Ansprüche, **dadurch gekennzeichnet, dass** die Zusammensetzung nach dem Trocknen einen Film bildet.

11. Zusammensetzung nach Anspruch 10, **dadurch gekennzeichnet, dass** der Film bei Raumtemperatur in reinem Wasser unlöslich ist.

12. Zusammensetzung nach Anspruch 10, **dadurch gekennzeichnet, dass** der Film bei einem sauren oder alkalischen pH-Wert löslich ist.

13. Zusammensetzung nach einem der vorhergehenden Ansprüche, **dadurch gekennzeichnet, dass** die grenzflächenaktiven Stoffe unter den anionischen, nichtionischen, amphoteren grenzflächenaktiven Stoffen und deren Gemischen ausgewählt sind.

14. Zusammensetzung nach Anspruch 13, **dadurch gekennzeichnet, dass** die anionischen grenzflächenaktiven Stoffe unter den Salzen der folgenden Verbindungen ausgewählt sind: Alkylsulfate, Alkylethersulfate, Alkylamidoethersulfate, Alkylarylpolyethersulfate, Monoglyceridsulfate; Alkylsulfonate, Alkylphosphate, Alkylamidsulfonate, Alkylarylsulfonate, α-Olefinsulfonate, Paraffinsulfonate; Alkylsulfosuccinate, Alkylethersulfosuccinate, Alkylamidsulfosuccinate; Alkylsulfoacetate; Acylsarcosinate; und Acylglutamate, wobei die Alkyl- oder Acylgruppen aller dieser Verbindungen 6 bis 24 Kohlenstoffatome aufweisen und die Arylgruppe vorzugsweise eine Phenyl- oder Benzylgruppe bedeutet; C₆₋₂₄-Alkylester von Polyglycosidcarbonsäuren; Alkylsulfosuccinamate, Acylisethionate und N-Acyltaurate verwendet werden, wobei die Alkyl- oder Acylgruppe 12 bis 20 Kohlenstoffatome aufweist; Acyllactylaten, deren Acylgruppe 8 bis 20 Kohlenstoffatome aufweist; Alkyl-D-galactosiduronsäuren und ihren Salzen sowie polyalkoxylierten Alkyl(C₆₋₂₄)ethercarbonsäuren, polyalkoxylierten Alkyl(C₆₋₂₄)aryl(C₆₋₂₄)ethercarbonsäuren, polyalkoxylierten Alkyl(C₆₋₂₄)amidoethercarbonsäuren und deren Salzen und deren Gemischen.

15. Zusammensetzung nach Anspruch 14, **dadurch gekennzeichnet, dass** die anionischen grenzflächenaktiven Stoffe unter den Salzen von Alkylsulfaten, Alkylethersulfaten und Alkylethercarboxylaten und deren Gemischen ausgewählt sind.

16. Zusammensetzung nach Anspruch 13, **dadurch gekennzeichnet, dass** die nichtionischen grenzflächenaktiven Stoffe ausgewählt sind unter den Alkoholen, Alphadiolen, Alkyl(C₁₋₂₀)phenolen oder C₈₋₁₈-Fettsäuren, die polyethoxyliert, polypropoxyliert oder mehrfach mit Glycerin verethert sind, wobei die Anzahl der Ethylenoxidgruppen oder Propylenoxidgruppen im Bereich von 2 bis 50 und die Anzahl der Glyceringruppen im Bereich von 2 bis 30 liegt, Copolymeren von Ethylenoxid und Propylenoxid, Kondensaten von Ethylenoxid und Propylenoxid mit Fettalkoholen; polyethoxylierten Fettamiden, die 2 bis 30 mol Ethylenoxid aufweisen, mehrfach mit Glycerin veretherten Fettamiden, die im Mittel 1 bis 5 Glyceringruppen enthalten; polyethoxylierten Fettaminen mit 2 bis 30 mol Ethylenoxid; ethoxylierten Sorbitanfettsäureestern mit 2 bis 30 mol Ethylenoxid; Saccharosefettsäureestern, Polyethylenglycolfettsäureestern, Alkyl(C₆₋₂₄)polyglycosiden, N-Alkyl(C₆₋₂₄)glucaminderivaten, Aminoxiden, und deren Gemischen.

17. Zusammensetzung nach Anspruch 13, **dadurch gekennzeichnet, dass** die amphoteren grenzflächenaktiven Stoffe unter den sekundären oder tertiären, aliphatischen Aminderivaten, bei denen die aliphatische Gruppe eine lineare Kette ist, die 8 bis 22 Kohlenstoffatome und mindestens eine wasserlösliche anionische Gruppe aufweist, wie Carboxylat, Sulfonat, Sulfat, Phosphat oder Phosphonat; Alkyl(C₈₋₂₀)betainen, Sulfonbetainen, Alkyl-(C₈₋₂₀)amidoalkyl(C₆₋₈)betainen oder Alkyl(C₈₋₂₀)amidoalkyl-(C₆₋₈)sulfobetainen; und deren Gemischen ausgewählt sind.

18. Zusammensetzung nach einem der vorhergehenden Ansprüche, **dadurch gekennzeichnet, dass** die Gesamtmenge des grenzflächenaktiven Stoffes oder der grenzflächenaktiven Stoffe im Bereich von 0,1 bis 30 Gew.-%, besser 1 bis 25 Gew.-% und noch besser 4 bis 20 Gew.-%, bezogen auf das Gesamtgewicht der Zusammensetzung, liegt.

19. Zusammensetzung nach einem der vorhergehenden Ansprüche, **dadurch gekennzeichnet, dass** sie ferner Zusatzstoffe enthält, wie natürliche oder synthetische, anionische, amphotere, zwitterionische, nichtionische oder kationische, assoziative oder nicht assoziative polymere Verdickungsmittel, nicht polymere Verdickungsmittel, wie Säuren oder Elektrolyte, Perlglanzstoffe, Trübungsmittel, organische Lösungsmittel, Parfums, mineralische, pflanzliche und/oder synthetische Öle, Fettsäureester, Farbmittel, flüchtige oder nicht flüchtige, organomodifizierte oder nicht organomodifizierte, cyclische oder acyclische, verzweigte oder nicht verzweigte Silicone, anorganische oder organische Partikel, Konservierungsmittel, pH-Stabilisatoren.

20. Verfahren zur kosmetischen Behandlung von Keratinsubstanzen, **dadurch gekennzeichnet, dass** auf die Keratinsubstanzen eine kosmetische Zusammensetzung aufgetragen wird, die in einem wässrigen Medium oder in Wasser + Lösungsmittel mindestens einen grenzflächenaktiven Stoff und mindestens eine nicht phosphorhaltige, kationische, verdickende molekulare Verbindung mit einer Molmasse unter 2 000 g/mol enthält, wobei die molekulare Verbindung mindestens aufweist:
- zwei Amidgruppen,
- ein asymmetrisches Kohlenstoffatom,
- eine Fettkette, die mindestens 8 Kohlenstoffatome enthält,
wobei unter einem kationischen Molekül ein Molekül zu verstehen ist, das mindestens ein quaternisiertes Stickstoffatom enthält
und **dadurch**, dass nach einer gegebenenfalls abgewarteten Einwirkzeit, gegebenenfalls gespült wird,.

21. Verfahren nach Anspruch 20, **dadurch gekennzeichnet, dass** die Keratinsubstanzen Haare sind.

22. Verwendung einer kosmetischen Zusammensetzung, die in einem wässrigen Medium oder in Wasser + Lösungsmittel mindestens einen grenzflächenaktiven Stoff und mindestens eine nicht phosphorhaltige, kationische, verdickende molekulare Verbindung mit einer Molmasse unter 2 000 g/mol enthält, wobei die molekulare Verbindung zumindest aufweist:
- zwei Amidgruppen,
- ein asymmetrisches Kohlenstoffatom,
- eine Fettkette mit mindestens acht Kohlenstoffatomen;
wobei unter einem kationischen Molekül ein Molekül verstanden wird, das mindestens ein quaternisiertes Stickstoffatom aufweist, um eine feuchtigkeitsbeständige Frisur zu gestalten.
